(19) Europäisches Patentamt
European Patent Office
Office européen des brevets

(11) EP 0 710 640 B1

## (12) EUROPEAN PATENT SPECIFICATION

(45) Date of publication and mention
of the grant of the patent:
**31.03.1999 Bulletin 1999/13**

(51) Int Cl.[6]: **C07C 29/80**, C07C 29/88,
C07C 29/132

(21) Application number: **95117236.0**

(22) Date of filing: **02.11.1995**

(54) **Recovery and purification of tertiary butyl alcohol**

Gewinnung und Reinigung von tertiärem Butylalkohol

Récupération et purification de l'alcool tertiobutylique

(84) Designated Contracting States:
**DE FR GB**

(30) Priority: **01.11.1994 US 332684**

(43) Date of publication of application:
**08.05.1996 Bulletin 1996/19**

(73) Proprietor: **Huntsman Specialty Chemicals
Corporation
Austin, Texas 78761 (US)**

(72) Inventors:
• **Chess, David D.
Houston, Texas 77057 (US)**
• **Sheu, Feng-Ran
Yorba Linda, CA 92686 (US)**
• **Kruse, Charles J.
Cypress, Texas 77429 (US)**

(74) Representative: **Goddar, Heinz J., Dr. et al
FORRESTER & BOEHMERT
Franz-Joseph-Strasse 38
80801 München (DE)**

(56) References cited:
**EP-A- 0 416 744** **US-A- 4 977 285**
**US-A- 5 254 759**

## Description

This invention relates to a method for the recovery of tertiary butyl alcohol from the reaction product formed by the catalytic reaction of propylene with tertiary butyl hydroperoxide in liquid phase in the presence of a soluble molybdenum catalyst.

More particularly, this invention relates to a method for recovering tertiary butyl alcohol from a higher boiling fraction of an epoxidation reaction product comprising tertiary butyl alcohol, unreacted tertiary butyl hydroperoxide, molybdenum catalyst and by-products formed by the removal of unreacted propylene and propylene oxide from an epoxidation product.

Prior Art

It is known to react propylene with tertiary butyl hydroperoxide in solution in tertiary butyl alcohol in the presence of a soluble molybdenum catalyst to form a reaction product comprising unreacted tertiary butyl hydroperoxide, unreacted propylene, propylene oxide, tertiary butyl alcohol, oxygen-containing by-products including methanol and acetone and dissolved molybdenum catalyst. This is shown, for example, in Kollar U.S. Patent No. 3,350,422; Kollar U.S. Patent No. 3,351,635; Marquis et al. U.S. Patent No. 4,845,251; and Marquis et al. U.S. Patent No. 4,891,437.

It is known to recover tertiary butyl hydroperoxide and tertiary butyl alcohol from an epoxidation product as described above, as shown, for example, in Marquis et al. U.S. Patent No. 4,992,566; and Marquis et al. U.S. Patent No. 4,977,285.

In the recovery sequence as shown in this group of Patents, tertiary butyl alcohol is recovered separately from a heavier fraction comprising tertiary butyl hydroperoxide, tertiary butyl alcohol and by-products.

In the recovery sequence disclosed in this group of patents, the heavy distillation fraction after further treatment will still contain unreacted tertiary butyl hydroperoxide and tertiary butyl alcohol which are recycled back to the epoxidation reaction zone for ultimate recovery.

It is also known that a tertiary butyl alcohol fraction containing minor amounts of tertiary butyl hydroperoxide can be treated to substantially completely decompose the tertiary butyl hydroperoxide and ditertiary butyl hydroperoxide contaminants by catalytic treatment in a separate reaction zone as shown, for example, by Sanderson et al. U. S. Patent No. 4,704,842; Sanderson et al. U. S. Patent No. 4,873,380; Sanderson et al. U. S. Patent No. 4,705,903; and Sanderson et al. U. S. Patent No. 4,704,482.

However, the processes disclosed in this group of Sanderson et al. patents require the use of a solid heterogeneous catalyst which is different from the molybdenum catalyst used for the epoxidation reaction.

Sanderson et al. also disclose the removal of tertiary butyl hydroperoxide contaminants from a tertiary butyl alcohol stream prepared by the catalytic decomposition of tertiary butyl hydroperoxide using a soluble homogeneous catalyst. Such processes are disclosed, for example, in Sanderson et al. U. S. Patent No. 4,910,349; Sanderson et al. U. S. Patent No. 4,912,266; Sanderson et al. U. S. Patent No. 4,912,267; Sanderson et al. U. S. Patent No. 4,922,033; Sanderson et al. U. S. Patent No. 4,922,034; Sanderson et al. U. S. Patent No. 4,922,035; Sanderson et al. U. S. Patent No. 4,992,602; and Sanderson et al. U. S. Patent No. 5,025,113.

Johnson U. S. Patent No. 3,825,605 discloses the liquid phase oxidation of an isoparaffin such as isobutane to the corresponding tertiary butyl alcohol in the presence of a homogeneous soluble molybdenum containing catalyst.

Grane U. S. Patent No. 3,474,151 discloses that contaminating quantities of tertiary butyl hydroperoxide contained in tertiary butyl alcohol can be decomposed thermally by heating the tertiary butyl alcohol at 190°C- 246°C (375°F-475°F) for from 1 to 10 minutes.

Background of the Present Invention

When propylene is reacted in liquid phase with tertiary butyl hydroperoxide in solution in tertiary butyl alcohol in the presence of a soluble epoxidation catalyst such as a molybdenum epoxidation catalyst, an epoxidation reaction product is formed comprising propylene oxide, tertiary butyl alcohol, unreacted propylene, unreacted tertiary butyl hydroperoxide, catalyst and impurities including water and other oxygenated impurities such as acetone and methanol.

In accordance with conventional practice, an epoxidation reaction product formed by the molybdenum-catalyzed reaction of propylene oxide with tertiary butyl hydroperoxide in solution in tertiary butyl alcohol is separated into the principle components by distillation so as to form distillation fractions including a propylene distillation fraction, a propylene oxide distillation fraction, a tertiary butyl alcohol distillation fraction and a heavy distillation fraction containing the molybdenum catalyst and other products and by-products of the epoxidation reaction. However, the distillation fractions that are thus-obtained are characterized by the inclusion of impurities and, normally, must be further treated if commercially acceptable products are to be obtained.

It has been discovered in accordance with the present invention that an enhanced yield of tertiary butyl alcohol substantially free from contaminating quantities of oxygen-containing by-products such as methanol and acetone can be provided through the provision of a process wherein unreacted propylene and propylene oxide are removed from the epoxidation reaction product to provide a higher boiling fraction which is distilled in a tertiary butyl alcohol removal column to provide a lighter tertiary butyl alcohol product substantially completely free from tertiary butyl hydroperoxide and containing only trace quantities of methanol and acetone and a higher boiling tertiary butyl hydroperoxide concentrate fraction comprising tertiary butyl alcohol, tertiary butyl hydroperoxide, molybdenum catalyst and impurities, the tertiary butyl hydroperoxide concentrate fraction being charged to a tertiary butyl hydroperoxide decomposition zone wherein the tertiary butyl hydroperoxide contained in the concentrate is substantially completely decomposed to provide additional tertiary butyl alcohol, oxygen and oxygen-containing products including methanol and acetone, the decomposition product being distilled in a tertiary butyl alcohol recovery column to provide an overhead distillation fraction comprising tertiary butyl alcohol contaminated with methanol and acetone and the higher boiling residue fraction comprising tertiary butyl alcohol, residual tertiary butyl hydroperoxide, molybdenum catalyst and impurities, and distilling the contaminated tertiary butyl alcohol fraction in a tertiary butyl alcohol purification column to obtain an overhead fraction comprising acetone and methanol and a higher boiling fraction consisting essentially of tertiary butyl alcohol which is substantially completely free from methanol and acetone.

In accordance with a preferred embodiment of the present invention, propylene is reacted with tertiary butyl hydroperoxide in solution in tertiary butyl alcohol in the presence of a soluble molybdenum catalyst to provide an epoxidation reaction product and unreacted propylene and propylene oxide are removed from the epoxidation product to provide a higher boiling distillation fraction.

In accordance with the present invention, the higher boiling distillation fraction is separated in a tertiary butyl alcohol removal column into a lighter tertiary butyl alcohol fraction containing only residual quantities of methanol and acetone and a higher boiling tertiary butyl hydroperoxide concentrate fraction comprising tertiary butyl alcohol, tertiary butyl hydroperoxide, molybdenum catalyst and impurities. The tertiary butyl hydroperoxide concentrate fraction is charged to a tertiary butyl hydroperoxide decomposition zone where the tertiary butyl hydroperoxide is substantially completely decomposed to provide additional tertiary butyl alcohol. The decomposition product that is thus formed is distilled in a tertiary butyl alcohol recovery column to provide an overhead contaminated tertiary butyl alcohol fraction containing methanol and acetone and a higher boiling residue fraction comprising tertiary butyl alcohol, tertiary butyl hydroperoxide, molybdenum catalyst and impurities, and the contaminated tertiary butyl alcohol fraction is charged to a tertiary butyl alcohol purification column where it is fractionated to provide a lighter distillation fraction comprising acetone and methanol and a higher boiling purified tertiary butyl alcohol fraction substantially completely free from methanol and acetone and consisting essentially of tertiary butyl alcohol.

In accordance with a preferred specific embodiment of the present invention, propylene and a tertiary butyl alcohol solution of tertiary butyl hydroperoxide and a soluble molybdenum catalyst are charged to an epoxidation reactor where the tertiary butyl hydroperoxide reacts with propylene to provide an epoxidation reaction product comprising unreacted propylene, unreacted tertiary butyl hydroperoxide, tertiary butyl alcohol, propylene oxide, soluble molybdenum catalyst and by-products including methanol and acetone,

wherein the epoxidation reaction product is distilled in a propylene removal distillation column under distillation conditions including an overhead temperature of 37°-65°C (100° to 150°F), a bottoms temperature of 104°- 127°C (220° to 260°F), and an operating pressure of 550-900 kPa (80 to 130 psia) to provide an overhead propylene recycle fraction and a higher boiling propylene free fraction,

wherein the higher boiling fraction is fractionated in a propylene oxide removal distillation column under distillation conditions including a top temperature of 32° to 49°C (90° to 120°F), and a bottoms temperature of 82° to 110°C (180° to 230°F) and an operating pressure of 104 to 208 kPa (15 to 30 psia) to provide a lighter distillation fraction comprising propylene oxide and a higher boiling fraction substantially free from propylene oxide and propylene,

wherein the higher boiling fraction is distilled in a tertiary butyl alcohol removal column to provide a lighter tertiary butyl alcohol fraction and a higher boiling tertiary butyl alcohol fraction comprising tertiary butyl alcohol, tertiary butyl hydroperoxide, molybdenum catalyst and impurities, the distillation conditions including a top temperature of 54° to 60°C (130° to 140°F), a bottom temperature of 93° to 104°C (200° to 220°F), and an operating pressure of 21 to 69 kPa (3 to 10 psia),

wherein the tertiary butyl hydroperoxide concentrate fraction is charged to a tertiary butyl hydroperoxide decomposition zone where the tertiary butyl hydroperoxide is catalytically decomposed under decomposition conditions including a temperature of 104° to 121°C (220° to 250°F), and a pressure of 345 to 690 kPa (50 to 100 psia) to provide a decomposition product comprising unreacted tertiary butyl hydroperoxide, tertiary butyl alcohol, catalyst and by-products including methanol and acetone,

wherein the decomposition product is distilled in a tertiary butyl alcohol recovery distillation column under distillation conditions including a top temperature of 54° to 60°C (130° to 140°F), a bottoms temperature of 115° to 132°C (240° to 270°F), and a pressure of 21 to 69 kPa (3 to 10 psia) to provide a lighter distillation fraction comprising tertiary butyl alcohol contaminated with methanol and acetone and a higher boiling residue fraction comprising tertiary butyl alcohol, tertiary butyl hydroperoxide, molybdenum catalyst and impurities,

wherein the contaminated tertiary butyl alcohol fraction is distilled in a tertiary butyl alcohol purification distillation column under distillation conditions including a top temperature of 66° to 77°C (150° to 170°F), a bottoms temperature of 93° to 110°C (200° to 230°F), and a pressure of 138 to 276 kPa (20 to 40 psia) to provide a lighter distillation fraction comprising methanol and acetone and a heavier distillation fraction consisting essentially of tertiary butyl alcohol substantially completely free from methanol and acetone.

## ACCOMPANYING DRAWING

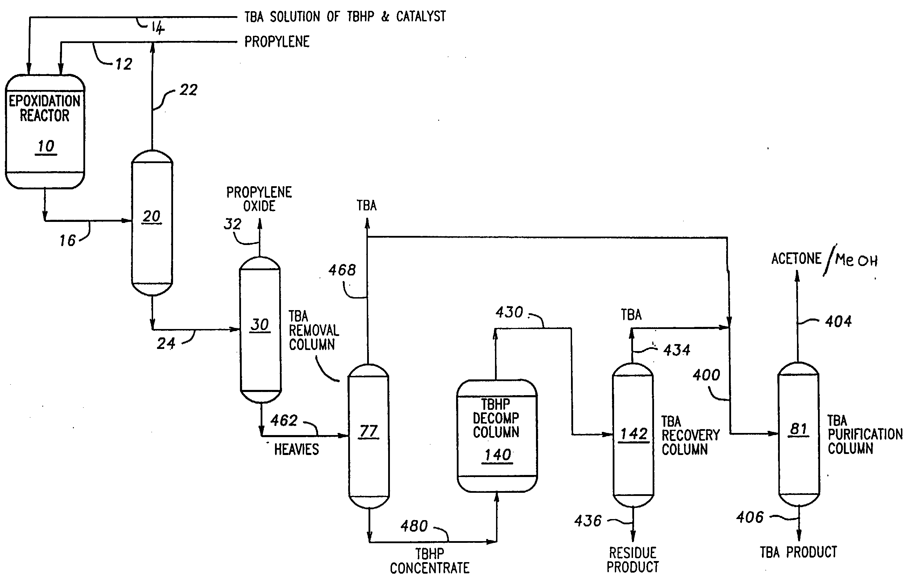

The Drawing is a schematic flow sheet with conventional parts omitted showing the general recovery sequence that is used in accordance with the present invention in obtaining purified tertiary butyl alcohol.

## DESCRIPTION OF THE PREFERRED EMBODIMENT

Turning now to the Drawing, there is shown a schematic flow sheet illustrating a preferred method for practising the process of the present invention. In the Drawing, conventional parts such as valves, pumps, temperature sensors, pressure sensors, heaters, coolers, flow control regulation apparatus, reboilers and reflux condensers have been omitted.

In accordance with the present invention, a solution of a soluble molybdenum catalyst solution (5 to 20 wt.% molybdenum) and a solution of tertiary butyl hydroperoxide (30 to 70 wt.%) is prepared. The tertiary butyl hydroperoxide and soluble molybdenum catalyst solutions are charged to an epoxidation reactor 10 by way of a charge line 14.

Propylene is also charged to the epoxidation reactor 10 by way of a charge line 12, the propylene being charged in an amount such that there is maintained in the epoxidation reactor 10 a ratio of 1.1 to 3 moles of propylene per mole of tertiary butyl hydroperoxide.

Epoxidation reaction conditions are established in the epoxidation reactor including a temperature within the range of 115° to 149°C (240° to 300°F), and more preferably within the range of 127° to 138°C (260° to 280°F). The catalyst concentration in the reactor 10 is preferably from 200 to 600 ppm. The reaction is conducted in the liquid phase at 3450 to 4830 kPa (500 to 700 psia). The concentration of tertiary butyl hydroperoxide in the tertiary butyl alcohol solution is suitably within the range of 30 to 70 wt.%.

The epoxidation reaction product formed in the epoxidation reactor 10 will comprise unreacted tertiary butyl hydroperoxide, unreacted propylene, tertiary butyl alcohol, propylene oxide, dissolved molybdenum catalyst and impurities including methanol and acetone.

The epoxidation product is discharged from the epoxidation reactor 10 by a line 16 leading to a propylene removal column 20 wherein the epoxidation reaction product 16 is separated into a lighter distillation fraction 22 which is suitably recycled to the propylene charge line 12 for the epoxidation reactor 10 and a heavier distillation fraction 24 substantially free from propylene which is charged to a propylene oxide recovery column 30 by way of line 24. Distillation conditions in the propylene removal column 20 will suitably include a top temperature of 38° to 66°C (100° to 150°F), a bottoms temperature of 104° to 127°C (220° to 260°F), and an operating pressure of 552 to 897 kPa (80 to 130 psia). Within the column 30, the feed is separated into a lighter distillation fraction comprising propylene oxide and small quantities of propylene which is discharged by a line 32 and a higher boiling fraction discharged from the column 30 by way of a line 462.

Distillation conditions in the propylene oxide removal column will suitably include a top temperature of 32° to 49°C (90° to 120°F), a bottoms temperature of 82° to 110°C (180° to 230°F), and an operating pressure of 104 to 207 kPa (15 to 30 psia).

The higher boiling fraction is fractionated in tertiary butyl alcohol removal column 77 under distillation conditions including a top temperature of 54° to 60°C (130° to 140°F), a bottoms temperature of 93° to 104°C (200° to 220°F), a pressure of 21 to 69 Kpa (3 to 10 psia) in order to provide a lighter distillation fraction separated by line 468 comprising principally of tertiary butyl alcohol and containing only trace quantities of acetone and methanol and a heavier tertiary butyl hydroperoxide concentrate fraction which is discharged from the tertiary butyl alcohol removal column 77 by a line 480 leading to a tertiary butyl hydroperoxide decomposition reactor or column 140. The tertiary butyl hydroperoxide concentrate will comprise tertiary butyl hydroperoxide, tertiary butyl alcohol, dissolved molybdenum catalyst and impurities. Tertiary butyl hydroperoxide decomposition reaction conditions are established in the tertiary butyl decomposition reactor 140 including a temperature of 104° to 121°C (220° to 250°F), and a pressure of 345 to 690 kPa (50 to 100 psia) to provide a decomposition product comprising unreacted tertiary butyl hydroperoxide, tertiary butyl alcohol, oxygen, dissolved molybdenum catalyst and oxygen-containing by-products including methanol and acetone. The tertiary butyl hydroperoxide decomposition product is charged by line 430 to a tertiary butyl alcohol recovery column 142 where it is separated under distillation conditions including a top temperature of 54° to 60°C (130° to 140°F), a bottoms temperature of 115° to 132°C (240° to 270°F) and a pressure of 21 to 69 kPa (3 to 10 psia) into a lighter contaminated tertiary butyl alcohol fraction 434 containing acetone and methanol and a heavier residue fraction 436 comprising tertiary butyl alcohol, unreacted tertiary butyl hydroperoxide, molybdenum catalyst and impurities.

The contaminated tertiary butyl alcohol fraction 434 is charged by a line 400 to a tertiary butyl alcohol purification column 81 wherein it is separated under distillation conditions including a top temperature of 66° to 77°C (150° to 170°F), a bottoms temperature of 93° to 110°C (200° to 230°F), and a pressure of 138 to 276 kPa (20 to 40 psia) into a lighter distillation fraction 404 comprising methanol and acetone and a heavier distillation fraction 406 consisting essentially of tertiary butyl alcohol and substantially completely free from methanol and acetone.

EXAMPLE

By way of example of the practice of the process of the present invention, a representative charge for the tertiary butyl alcohol removal column 77 may have the composition shown in Table 1.

Table 1

| | Weight % |
|---|---|
| Acetone | .05 |
| Methanol | .07 |
| Tertiary Butyl Alcohol | 90.70 |
| Formic Acid | .47 |
| Tertiary Butyl Hydroperoxide | 1.20 |
| Heavies | 7.51 |
| | 100.00 |

When utilizing distillation conditions including a top temperature of 54° to 60°C (130° to 140°F), a bottoms temperature of 93° to 104°C (200° to 220°F), and an operating pressure of 21 to 69 kPa (3 to 10 psia), the tertiary butyl alcohol fraction 468 may be obtained containing, for example, 0.06 wt.% acetone, 0.08 wt.% methanol and 98.0 wt.% tertiary butyl alcohol and 1 ppm of tertiary butyl hydroperoxide. Thus, the tertiary butyl alcohol fraction is of a very high degree of purity containing less than about two-tenths of one percent of acetone, methanol, and tertiary butyl hydroperoxide.

The higher boiling tertiary butyl hydroperoxide concentrate fraction 480 withdrawn from the tertiary butyl alcohol removal column 77 will have, for example, the composition shown in Table 2.

Table 2

| | Weight % |
|---|---|
| Tertiary Butyl Alcohol | 26.29 |
| Formic Acid | 4.78 |
| Tertiary Butyl Hydroperoxide | 12.26 |
| Heavies | 56.87 |
| | 100.00 |

The tertiary butyl hydroperoxide concentrate fraction 480 is charged to a tertiary butyl hydroperoxide decomposition reaction zone 140, such as a column, where tertiary butyl hydroperoxide decomposition conditions are established including a temperature of 104° to 121°C (220° to 250°F) and a pressure of 345 to 690 kPa (50 to 100 psia) to bring about the substantial decomposition of the tertiary butyl hydroperoxide contained in the fraction.

Thus, the tertiary butyl hydroperoxide decomposition product discharged by the line 430 may have a composition as shown in Table 3.

Table 3

| | Weight % |
|---|---|
| Acetone | 1.15 |
| Methanol | 0.85 |
| Tertiary Butyl Alcohol | 35.09 |
| Formic Acid | 4.78 |
| Tertiary Butyl Hydroperoxide | 1.26 |
| Heavies | 56.87 |
| | 100.00 |

It will be seen, therefore, that substantially all of the tertiary butyl hydroperoxide was decomposed in the decomposition zone 140 and that among the decomposition products that were formed were tertiary butyl alcohol, oxygen, acetone and methanol.

The tertiary butyl hydroperoxide decomposition product is charged to a tertiary butyl alcohol recovery column 142 which is operated under distillation conditions including a top temperature of 54° to 60°C (130° to 140°F), a bottoms temperature of 115° to 132°C (240° to 270°F), and an operating pressure of 21 to 69 kPa (3 to 10 psia). The decomposition product is separated in the tertiary butyl alcohol recovery column 142 into a lighter contaminated tertiary butyl alcohol fraction 434 containing, for example, 3.2 wt.% acetone, 2.1 wt.% methanol and 92.2 wt.% tertiary butyl alcohol.

A higher boiling residue fraction 436 is discharged from the tertiary butyl alcohol recovery column 142 which may, for example, have the composition shown in table 4.

Table 4

| | Weight % |
|---|---|
| Tertiary Butyl Alcohol | 3.10 |
| Formic Acid | 7.39 |
| Tertiary Butyl Hydroperoxide | 2.74 |
| Heavies | 86.77 |
| | 100.00 |

It will be seen from this that only a minor amount of tertiary butyl alcohol was lost to the process in the fraction 436 and that the residual tertiary butyl hydroperoxide, formic acid and heavies were substantially quantitatively removed. The heavies fraction 436 will, of course, contain the soluble molybdenum catalyst.

The contaminated tertiary butyl alcohol fraction 434 is charged to a tertiary butyl alcohol purification column 81 which is operated under distillation conditions including a top temperature of 66° to 77°C (150° to 170°F), a bottoms temperature of 93° to 110°C (200° to 230°F), and a pressure of 138 to 276 kPa (20 to 40 psia) to provide a lighter impurities fraction 404 comprising substantially all of the acetone and methanol charged to the tertiary butyl alcohol purification column 81 and a higher boiling purified tertiary butyl alcohol fraction containing substantially all of the tertiary butyl alcohol charged to the tertiary butyl alcohol purification column 81.

**Claims**

1. A method for the preparation of tertiary butyl alcohol and propylene oxide by the reaction of propylene with tertiary butyl hydroperoxide in the presence of a soluble molybdenum catalyst to provide an epoxidation reaction product comprising unreacted propylene, propylene oxide, tertiary butyl alcohol, unreacted tertiary butyl hydroperoxide, molybdenum catalyst and by-products, which comprises:

distilling said epoxidation reaction product (16) to remove unreacted propylene (22) and propylene oxide (32) and to obtain a higher boiling fraction (462);

distilling said higher boiling fraction (462) of the reaction product in a tertiary butyl alcohol removal distillation column (77) into an overhead tertiary butyl alcohol fraction (468) and a higher boiling tertiary butyl hydroperoxide concentrate fraction (480) comprising tertiary butyl alcohol, tertiary butyl hydroperoxide, molybdenum catalyst and impurities;

charging said tertiary butyl hydroperoxide concentrate fraction (480) to a tertiary butyl hydroperoxide decomposition zone (140) and catalytically decomposing the tertiary butyl hydroperoxide contained therein to provide a decomposition product (430) comprising unreacted tertiary butyl hydroperoxide, tertiary butyl alcohol, oxygen, and oxygen-containing by-products including methanol and acetone;

distilling said decomposition product (430) in a tertiary butyl alcohol recovery distillation column (142) into an overhead contaminated recovered tertiary butyl alcohol fraction (434) containing methanol and acetone and a higher boiling residue fraction (436) comprising tertiary butyl alcohol, tertiary butyl hydroperoxide, molybdenum catalyst and impurities;

distilling said recovered contaminated tertiary butyl alcohol fraction in a tertiary butyl alcohol purification distillation column (61) into an overhead impurities fraction (404) comprising acetone and methanol and a higher boiling purified tertiary butyl alcohol fraction (406) ; and

revocering said higher boiling purified tertiary butyl alcohol fraction (406).

2. A method as in claim 1 which comprises:

distilling said higher boiling fraction in a tertiary butyl alcohol removal distillation column (77) under distillation conditions including a top temperature of 54° to 60°C (130° to 140°F), a bottom temperature of 93° to 104°C. (200° to 220°F), and an operating pressure of 21 to 69 kPa (3 to 10 psia) to obtain a tertiary butyl hydroperoxide concentrate fraction;

charging said tertiary butyl hydroperoxide concentrate fraction to a tertiary butyl hydroperoxide decomposition zone (140) and catalytically decomposing the tertiary butyl hydroperoxide contained therein under tertiary butyl hydroperoxide decomposition conditions including a temperature of 104° to 121°C, (220° to 250°F), and an operating pressure of 345 to 690 kPa (50 to 100 psia) to form a decomposition product (430);

distilling said decomposition product (430) in a tertiary butyl alcohol recovery distillation column (142) under distillation conditions including a top temperature of 54° to 60°C. (130° to 140°C), a bottoms temperature of 115° to 132°C (240° to 270°F), and an operating pressure of 21 to 69 kPa (3 to 10 psia) to recover a contaminated tertiary butyl alcohol fraction (434); and

distilling said recovered contaminated tertiary butyl alcohol fraction (434) in a tertiary butyl alcohol purification distillation column (81) under distillation conditions including a top temperature of 66° to 77°C (150° to 170°F), a bottoms temperature of 93° to 110°C (200° to 230°F), and an operating pressure of 138 to 276 kPa (20 to 40 psia).

3. A method as in claim 1 which comprises:

distilling said epoxidation reaction product in a propylene removal distillation column (20) under distillation conditions including a top temperature of 38° to 66°C (100° to 150°F) a bottoms temperature of 104° to 127°C (220° to 260°F), and an operating pressure of 552 to 897 kPa (80 to 130 psia), into an overhead propylene rich fraction (22) and a higher boiling fraction (24) comprising propylene, propylene oxide, tertiary butyl alcohol, tertiary butyl hydroperoxide, molybdenum catalyst and impurities;

distilling said higher boiling fraction (24) in an propylene oxide removal distillation column (30) under distillation conditions including a top temperature of 32° to 49°C (90° to 120°F), a bottoms temperature of 82° to 121°C (220° to 250°F), and an operating pressure of 104 to 207 kPa (15 to 30 psia), into an overhead propylene oxide rich fraction (32) and a higher boiling propylene oxide free fraction (462) comprising tertiary butyl alcohol, tertiary butyl hydroperoxide, molybdenum catalyst and impurities; and

distilling said higher boiling fraction (462) in a tertiary butyl alcohol removal distillation column (77) under distillation conditions including a top temperature of 54° to 60°C (130° to 140°F), a bottoms temperature of 93° to 104°C (200° to 220°F), and an operating pressure of 21 to 69 kPa (3 to 10 psia), into an overhead tertiary butyl alcohol fraction (468) and a higher boiling tertiary butyl hydroperoxide concentrate fraction (480) comprising tertiary butyl alcohol, tertiary butyl hydroperoxide, molybdenum catalyst and impurities.

## Patentansprüche

1. Ein Verfahren zur Herstellung von tert.-Butylalkohol und Propylenoxid durch die Reaktion von Propylen mit tert.-Butylhydroperoxid in der Gegenwart eines löslichen Molybdänkatalysators, um ein Epoxidationsreaktionsprodukt zu liefern, das nicht-umgesetztes Propylen, Propylenoxid, tert.-Butylalkohol, nicht-umgesetztes tert.-Butylhydroperoxid, Molybdänkatalysator und Nebenprodukte umfaßt, welches umfaßt:

Destillieren besagten Epoxidationsreaktionsproduktes (16), um nicht-umgesetztes Propylen (22) und Propylenoxid (32) zu entfernen und eine höher siedende Fraktion (462) zu erhalten;

Destillieren besagter höhersiedenden Fraktion (462) des Reaktionsproduktes in einer Destillationskolonne zur Entfernung von tert.-Butylalkohol (77) in eine Überkopf-tert.-Butylalkoholfraktion (468) und eine höhersiedende tert.-Butylhydroperoxid-Konzentratfraktion (480), die tert.-Butylalkohol, tert.-Butylhydroperoxid, Molybdänkatalysator und Verunreinigungen umfaßt;

Zuführen besagter tert.-Butylhydroperoxid-Konzentratfraktion (480) zu einer tert.-Butylhydroperoxid-Zersetzungszone (140) und katalytisches Zersetzen des darin enthaltenen tert.-Butylhydroperoxids, um ein Zersetzungsprodukt (430) zu liefern, das nicht-umgesetztes tert.-Butylhydroperoxid, tert.-Butylalkohol, Sauerstoff und sauerstoffhaltige Nebenprodukte, einschließlich Methanol und Aceton, umfaßt;

Destillieren besagten Zersetzungsproduktes (430) in einer Destillationskolonne zur Gewinnung von tert.-Butylalkohol, (142) in eine verunreinigte, rückgewonnene Überkopf-tert.-Butylalkoholfraktion (434), die Methanol und Aceton enthält, und eine höhersiedende Rückstandsfraktion (436), die tert.-Butylalkohol, tert.-Butylhydroperoxid, Molybdänkatalysator und Verunreinigungen umfaßt;

Destillieren besagter rückgewonnener verunreinigter tert.-Butylalkoholfraktion in einer Destillationskolonne zur Reinigung von tert.-Butylalkohol (61) in eine Überkopf-Verunreinigenfraktion (404), die Aceton und Methanol umfaßt, und eine höhersiedende gereinigte tert.-Butylalkoholfraktion (406); und

Gewinnen besagter höhersiedenden gereinigten tert.-Butylalkoholfraktion (406).

2. Ein Verfahren nach Anspruch 1, welches umfaßt:

Destillieren besagter höhersiedenden Fraktion in einer Destillationskolonne zur Entfernung von tert.-Butylalkohol (77) unter Destillationsbedingungen, die eine Kopftemperatur von 54° bis 60°C (130° bis 140°F), eine Bodentemperatur von 93° bis 104°C (200° bis 220°F) und einen Betriebsdruck von 21 bis 69 kPa (3 bis 10 psia) einschließen, um eine tert.-Butylhydroperoxid-Konzentratfraktion zu erhalten;

Zuführen besagter tert.-Butylhydroperoxid-Konzentratfraktion zu einer tert.-Butylhydroperoxidzersetzungszone (140) und katalytisches Zersetzen des darin enthaltenen tert.-Butylhydroperoxids unter tert.-Butylhydroperoxid-Zersetzungsbedingungen, die eine Temperatur von 104° bis 121°C (220° bis 250°F) und einen Betriebsdruck von 345 bis 690 kPa (50 bis 100 psia) einschließen, um ein Zersetzungsprodukt (430) zu bilden;

Destillieren besagten Zersetzungsproduktes (430) in einer Destillationskolonne zur Gewinnung von tert.-Butylalkohol (142) unter Destillationsbedingungen, die eine Kopftemperatur von 54° bis 60°C (130° bis 140°F), eine Bodentemperatur von 115° bis 132°C (240° bis 270°F) und einen Betriebsdruck von 21 bis 69 kPa (3 bis 10 psia) einschließen, um eine verunreinigte tert.-Butylalkoholfraktion (434) zu gewinnen; und

Destillieren besagter gewonnenen verunreinigten tert.-Butylalkoholfraktion (434) in einer Destillationskolonne zur Reinigung von tert.-Butylalkohol (81) unter Destillations-bedingungen, die eine Kopftemperatur von 66° bis 77°C (150° bis 170°F), eine Bodentemperatur von 93° bis 110°C (200° bis 230°F) und einen Betriebsdruck von 138 bis 276 kPa (20 bis 40 psia) einschließen.

3. Ein Verfahren nach Anspruch 1, welches umfaßt:

Destillieren besagten Epoxidationsreaktionsproduktes in einer Destillationskolonne zur Entfernung von Propylen (20) unter Destillationsbedingungen, die eine Kopftemperatur von 38° bis 66°C (100° bis 150°F), eine Bodentemperatur von 104° bis 127°C (220° bis 260°F) und einen Betriebsdruck von 552 bis 897 kPa (80 bis 130 psia) einschließen, in eine an Propylen reiche Überkopffraktion (22) und eine höhersiedende Fraktion (24), die Propylen, Propylenoxid, tert.-Butylalkohol, tert.-Butylhydroperoxid, Molybdänkatalysator und Verunreinigungen umfaßt;

Destillieren besagter höhersiedenden Fraktion (24) in einer Destillationskolonne zur Entfernung von Propylenoxid (30) unter Destillationsbedingungen, die eine Kopftemperatur von 32° bis 49°C (90° bis 120°F), eine Bodentemperatur von 82° bis 110°C (180° bis 230°F) und einen Betriebsdruck von 104 bis 207 kPa (15 bis 30 psia) einschließen, in eine an Propylenoxid reiche Überkopffraktion (32) und eine höhersiedende, von Propylenoxid freie Fraktion (462), die tert.-Butylalkohol, tert.-Butylhydroperoxid, Molybdänkatalysator und Verunreinigungen umfaßt; und

Destillieren besagter höhersiedenen Fraktion (462) in einer Destillationskolonne zur Entfernung von tert.-Butylalkohol (77) unter Destillationsbedingungen, die eine Kopftemperatur von 54° bis 60°C (130° bis 140°F), eine Bodentemperatur von 93° bis 104°C (200° bis 220°F) und einen Betriebsdruck von 21 bis 69 kPa (3 bis 10 psia) einschließen, in eine Überkopftert.-Butylalkoholfraktion (468) und eine höhersiedende tert.-Butylhydroperoxid-Konzentratfraktion (480), die tert.-Butylalkohol, tert.-Butylhydroperoxid, Molybdänkatalysator und Verunreinigungen umfaßt.

## Revendications

1. Procédé de préparation d'alcool tertiobutylique et d'oxyde de propylène par la réaction de propylène avec de l'hydroperoxyde tertiobutylique, en présence d'un catalyseur au molybdène soluble, pour fournir un produit de réaction d'époxydation comprenant du propylène n'ayant pas réagi, de l'oxyde de propylène, de l'alcool tertiobutylique, de l'hydroperoxyde tertiobutylique n'ayant pas réagi, du catalyseur au molybdène et des sous-produits, qui comprend :

la distillation dudit produit de réaction d'époxydation (16) pour éliminer le propylène n'ayant pas réagi (22) et l'oxyde de propylène (32) et pour obtenir une fraction à point d'ébullition plus élevé (462) ;
la distillation de ladite fraction à point d'ébullition plus élevé (462) du produit de réaction, dans une colonne de distillation (77) d'élimination de l'alcool tertiobutylique, en une fraction de tête d'alcool tertiobutylique (468) et une fraction concentrée à point d'ébullition plus élevé d'hydroperoxyde tertiobutylique (480) comprenant de l'alcool tertiobutylique, de l'hydroperoxyde tertiobutylique, du catalyseur au molybdène et des impuretés ;
le chargement de ladite fraction concentrée d'hydroperoxyde tertiobutylique (480) dans une zone de décomposition (140) de l'hydroperoxyde tertiobutylique et la décomposition catalytique de l'hydroperoxyde tertiobutylique contenu dedans, pour fournir un produit de décomposition (430) comprenant de l'hydroperoxyde tertiobutylique n'ayant pas réagi, de l'alcool tertiobutylique, de l'oxygène et des sous-produits contenant de l'oxygène incluant du méthanol et de l'acétone ;
la distillation dudit produit de décomposition (430) dans une colonne de distillation de récupération de l'alcool tertiobutylique (142), en une fraction de tête d'alcool tertiobutylique récupérée contaminée (434), contenant du méthanol et de l'acétone et en une fraction de résidus à point d'ébullition plus élevé (436) comprenant de l'alcool tertiobutylique, de l'hydroperoxyde tertiobutylique, du catalyseur au molybdène et des impuretés ;
la distillation de ladite fraction d'alcool tertiobutylique contaminée récupérée dans une colonne de distillation de purification de l'alcool tertiobutylique (81) en une fraction de tête d'impuretés (404) comprenant de l'acétone et du méthanol et en une fraction purifiée, à point d'ébullition plus élevé d'alcool tertiobutylique (406) ; et

la récupération de ladite fraction purifiée, à point d'ébullition plus élevé d'alcool tertiobutylique (406).

2. Procédé selon la revendication 1 qui comprend :

la distillation de ladite fraction à point d'ébullition plus élevé dans une colonne de distillation (77) d'élimination de l'alcool tertiobutylique, sous des conditions de distillation incluant une température de tête de 54° à 60°C (130° à 140°F), une température de fond de 93° à 104°C (200° à 220°F), et une pression de fonctionnement de 21 à 69 kPa (3 à 10 psia) pour obtenir une fraction concentrée d'hydroperoxyde tertiobutylique ;

le chargement de ladite fraction concentrée d'hydroperoxyde tertiobutylique dans une zone de décomposition (140) de l'hydroperoxyde tertiobutylique et la décomposition catalytique de l'hydroperoxyde tertiobutylique contenu dedans sous des conditions de décomposition de l'hydroperoxyde tertiobutylique incluant une température de 104° à 121°C (220° à 250°F), et une pression de fonctionnement de 345 à 690 kPa (50 à 100 psia) pour former un produit de décomposition (430);

la distillation dudit produit de décomposition (430) dans une colonne de distillation de récupération de l'alcool tertiobutylique (142), sous des conditions de distillation incluant une température de tête de 54° à 60°C (130° à 140°F), une température de fond de 115° à 132°C (240° à 270°F), et une pression de fonctionnement de 21 à 69 kPa (3 à 10 psia) pour récupérer une fraction contaminée d'alcool tertiobutylique (434) ; et

la distillation de ladite fraction d'alcool tertiobutylique contaminée récupérée (434) dans une colonne de distillation de purification de l'alcool tertiobutylique (81), sous des conditions de distillation incluant une température de tête de 66° à 77°C (150° à 170°F), une température de fond de 93° à 110°C (200° à 230°F) et une pression de fonctionnement de 138 à 276 kPa (20 à 40 psia).

3. Procédé selon la revendication 1 qui comprend :

la distillation dudit produit de réaction d'époxydation dans une colonne de distillation (20) d'élimination du propylène, sous des conditions de distillation incluant une température de tête de 38° à 66°C (100° à 150°F), une température de fond de 104° à 127°C (220° à 260°F) et une pression de fonctionnement de 552 à 897 kPa (80 à 130 psia), en une fraction de tête enrichie en propylène (22) et une fraction à point d'ébullition plus élevé (24) comprenant du propylène, de l'oxyde de propylène, de l'alcool tertiobutylique, de l'hydroperoxyde tertiobutylique, du catalyseur au molybdène et des impuretés ;

la distillation de ladite fraction à point d'ébullition plus élevé (24) dans une colonne de distillation d'élimination de l'oxyde de propylène (30), sous des conditions de distillation incluant une température de tête de 32° à 49°C (90° à 120°F), une température de fond de 82° à 110°C (180° à 230°F) et une pression de fonctionnement de 104 à 207 kPa (15 à 30 psia), en une fraction de tête enrichie en oxyde de propylène (32) et une fraction à point d'ébullition plus élevé exempt d'oxyde de propylène (462) comprenant de l'alcool tertiobutylique, de l'hydroperoxyde tertiobutylique, du catalyseur au molybdène et des impuretés ; et

la distillation de ladite fraction à point d'ébullition plus élevé (462) dans une colonne de distillation d'élimination de l'alcool tertiobutylique (77), sous des conditions de distillation incluant une température de tête de 54° à 60°C (130° à 140°F), une température de fond de 93° à 104°C (200° à 220°F) et une pression de fonctionnement de 21 à 69 kPa (3 à 10 psia), en une fraction de tête d'alcool tertiobutylique (468) et une fraction concentrée à point d'ébullition plus élevé d'hydroperoxyde tertiobutylique (480) comprenant de l'alcool tertiobutylique, de l'hydroperoxyde tertiobutylique, du catalyseur au molybdène et des impuretés.

TBA SOLUTION OF TBHP & CATALYST

PROPYLENE

14

12

22

EPOXIDATION REACTOR

10

20

16

PROPYLENE OXIDE

32

TBA

468

24

30

TBA REMOVAL COLUMN

462

HEAVIES

77

480

TBHP CONCENTRATE

TBHP DECOMP COLUMN

140

430

434

TBA

400

TBA RECOVERY COLUMN

142

436

RESIDUE PRODUCT

ACETONE / MeOH

404

TBA PURIFICATION COLUMN

81

406

TBA PRODUCT